# EUROPEAN PATENT APPLICATION

(11) **EP 4 070 784 A1**
(43) Date of publication of application: **12.10.2022**
(21) Application number: 19955375.1
(22) Date of filing: 06.12.2019
(51) Int. Cl.: A61K 9/00, C12N 15/00, C12N 13/00, B01D 71/70, A61L 27/44, C08J 3/075, B01J 13/00

(54) **COMPOSITE MATERIAL AND PREPARATION METHOD THEREFOR AND APPLICATION THEREOF**

(71) Applicant: Biomodi Biotech (Suzhou) Co., Ltd, Suzhou, Jiangsu 215127 (CN)
(72) Inventor: CHEN, Hong, Suzhou, Jiangsu 215123 (CN); WANG, Lei, Suzhou, Jiangsu 215123 (CN); TANG, Heming, Suzhou, Jiangsu 215123 (CN)
(74) Representative: Klöckner, Christoph
(86) International application number: PCT/CN2019/123703
(87) International publication number: WO 2021/109132

(57) **Abstract**

The present disclosure provides a composite material. The composite material comprises nanoparticles and a flexible substrate, the nanoparticles comprise one or more of carbon nanotubes, graphene, gold nanoparticles, and polydopamine nanoparticles, the flexible substrate comprises one or more of thermosetting plastics such as polydimethylsiloxane and a hydrogel, and the mass percentage of the nanoparticles in the composite material is 0 to 60‰. The composite material of the present disclosure is easy to prepare, has extremely strong photothermal conversion performance, and does not change the smooth surface of an original topological structure. Meanwhile, the composite material has universality and versatility for different cells, the delivery efficiency is close to 100%, and modified cells may be efficiently and non-destructively released and harvested by means of traditional trysinization, and the harvesting efficiency is 90% or more.

## Description

### TECHNICAL FIELD

The present disclosure relates to a composite material, and specifically to a composite material comprising nanoparticles and a flexible substrate. The present disclosure also relates to a preparation method for the above composite material and the use of the composite material in intracellular macromolecule delivery.

### BACKGROUND

Nanoparticles have unique optical, electrical, and catalytic properties due to their nanoscale size. Some nanoparticles (e.g., metal nanoparticles such as Au, metal oxide nanoparticles such as ferroferric oxide, and polymer nanoparticles such as polydopamine) may absorb a large amount of laser energy and convert it into thermal energy due to their excellent surface plasmon resonance effect. Therefore, nanoparticles are widely used in fields such as tumor photothermal therapy and intracellular macromolecule delivery via photoporation in vivo and in vitro.

The technology of intracellular macromolecule delivery is able to endow cells with specific properties by introducing functional exogenous macromolecules, which is the key to carrying out many biomedical research and clinical applications. Currently, the commonly used macromolecule delivery methods mainly include the vector method (including viral vectors, liposomes, or polymer vectors) and the physical method of membrane rupture. Among them, although the viral vector method has high efficiency, its poor safety and the only delivery of nucleic acid limit its further application. When delivering nucleic acid, most viruses will integrate their own DNA into the host cells, resulting in gene mutation of the host cells, and serious ones may even lead to canceration of modified cells after implanting into the body, which is harmful to human health. Therefore, the transfection of cells by viral vector method is difficult to be widely used in clinical medicine. However, the vector methods of liposome and polymer are difficult to be used on a large scale because of their low delivery efficiency and strong cytotoxicity.

Compared with the technology of the delivery of exogenous macromolecules by the vector method, the technology of the delivery of exogenous macromolecules by the physical method of membrane rupture has been paid more and more attention because of its high delivery efficiency, low cytotoxicity, strong universality, and overcoming the shortcomings such as strong infectivity and low safety of traditional viral vectors. According to different ways of membrane rupture, the physical methods of membrane rupture are mainly classified into the mechanical method of membrane rupture and the electromagnetic/thermal method of membrane rupture, including multiple directions such as cell extrusion of membrane rupture (Document 1), microinjection (Document 2), electroporation method (Document 3), and photothermal method of membrane rupture (Document 4). Among them, the mechanical method of membrane rupture mainly acts on the cell through the external force so that the cell membrane occurs reversible damage to deliver exogenous macromolecules to the cell. Document 1 shows that squeezing cells through microfluidic channels may produce modified cells on a large scale, but the equipment is complex, and expensive and the delivery efficiency of exogenous macromolecules is low. Document 2 discloses a microinjection device, and the microinjection device has high delivery efficiency, but may only operate on single cells, has low efficiency, high labor and equipment costs, and is not conducive to practical clinical application. In summary, the mechanical method of membrane rupture mostly requires complex instruments or devices, the experimental threshold is high, the experimental price is expensive, and the artificial requirements are too high, which does not meet the needs of actual clinical treatment to obtain a large number of modified cells as cheap, convenient, fast, efficient and safe as possible.

Compared with the mechanical method of membrane rupture, the electromagnetic/thermal method of membrane rupture only changes the external electric, magnetic, light, thermal field without direct interaction with cells, thus reducing the cell damage and being easier to operate than that of the mechanical method of membrane rupture. Therefore, this method has been paid more and more attention in recent years. Electroporation is one of the most common electromagnetic/thermal methods of membrane rupture, which has high delivery efficiency. However, the intensity of the high-voltage electric pulse used in the delivery process is high, so that the cell viability is significantly reduced, and the effect of cell adherence and expression after harvesting is not ideal, which limits its promotion and application. Yet, the macromolecule delivery via photoporation is mainly achieved through laser irradiation of nanoparticles, using the unique photothermal conversion ability of nanoparticles to heat the surrounding tissue or cells, making the cell membrane instantly reversible perforation, thereby improving the permeability of the cell membrane for intracellular macromolecule delivery. Compared with other macromolecule delivery methods, the photoporation method has not only simple operation and large cell throughput but also has strong versatility of cells and macromolecules, so this method is a very promising means of living cell macromolecule delivery. The conventional photoporation platform delivers macromolecules intracellularly by nanoparticles as photoporation reagent. Although the operation is simple, it also has obvious drawbacks: 1. nanoparticles are easy to enter cells through endocytosis, and most nanoparticles may not be degraded through normal metabolism, thus they have certain cytotoxicity to cells; 2. decreasing the concentration of nanoparticles may moderately alleviate the cytotoxicity, but it will reduce the photothermal efficiency, and ultimately lead to the reduction of intracellular macromolecule delivery efficiency.

Based on nanoparticles, in order to reduce the cytotoxicity of nanoparticles and improve the delivery efficiency of intracellular macromolecule delivery, Document 4 discloses an intracellular exogenous macromolecules delivery platform with a substrate surface modified by a gold nanoparticle layer with a photothermal effect. The platform enables the photothermal nanoparticles to be enriched on the substrate surface through surface modification. Compared with nanoparticles, the enriched gold nanoparticle layer is not easy to be endocytosed by cells, which improves the cytotoxicity of photothermal materials, and the photothermal efficiency is improved, so that a low-intensity laser light source may be used for macromolecule delivery, and the influence on cell viability is reduced while the delivery efficiency is improved. However, the topological structure changes of the substrate surface caused by the surface modified nanoparticle layer bring inevitable side effects, which makes it difficult to harvest the modified cells from the substrate for further research and application. In order to solve the side effects of surface-modified nanoparticle layer, Document 5 shows that a method of combining the photoporation for gold nanoparticle with liposome, and this method successfully transfect difficult-to-transfect cells with high efficiency. However, it is difficult to harvest the modified cells from the substrate for further research and application due to the topological structure of the material surface and the easy phagocytosis of the cells. In Document 6, polydopamine nanoparticles are used as a photothermal substrate to modify the temperature-sensitive polymer PNIPAAM on the surface, and the release of cells is controlled by temperature change. Yet, the preparation temperature has significant influence on the surface modification of the temperature-sensitive polymer so that the repeatability of the material is poor, the surface modification operation of the material is complex, the time consumption is long, and the shelf life of the prepared material is short. Moreover, the polymer is modified with only one layer of polymer brush so that the temperature sensitivity is insufficient, the temperature control of cell release is not easy to operate, the cells are easily damaged, and the like, the efficiency of actually harvesting effective cells with good viability is very low, and the problem of cell harvesting in photothermal transfection is not really solved; thus, the photoporation method is difficult to be practically applied on a large scale. In Document 7, silicon nanowires are used as photothermal substrates; by modifying the surface with sugar-responsive phenylboronic acid and using nontoxic natural biomolecules (such as sugar) as stimuli, the cell release may be triggered under mild conditions, but high-viability cells may still not be efficiently harvested.

In summary, at present, photothermal intracellular macromolecule delivery platforms based on nanoparticle particles and nanoparticle layers are still unable to meet the practical application needs. Therefore, the design of a macromolecular delivery system with low cost, simple materials, convenient and fast operation, strong universality of exogenous molecules and cell types, high delivery efficiency, low cytotoxicity, and large throughput for treating cells, high cell harvesting rate, and good cell viability remains to be studied.
Document 1. Integr. Biol, 2014, 6, 470--475;
Document 2. CN105420099B;
Document 3. CN105143436B;
Document 4. CN105420278A;
Document 5. ACS Appl. Mater. Interfaces, 2017, 9, 21593-21598;
Document 6. ACS Appl. Mater. Interfaces, 2019, 11, 12357-12366;
Document 7. Adv. Funct. Mater, 2019, 1906362.

### SUMMARY

### Technical problem

In order to overcome the above technical problems, the present disclosure provides a composite material with low cost, simple materials, convenient and fast operation, strong universality of exogenous molecules and cell types, high delivery efficiency, low cytotoxicity, and large throughput for treating cells, high cell harvesting rate, and good cell viability.

### Solution to the problems

In order to solve the technical problem of the present disclosure, the present disclosure provides the following technical solutions:
A composite material, wherein the composite material comprises nanoparticles and a flexible substrate, the nanoparticles comprise one or more of photothermal nanoparticles such as carbon nanotubes, graphene, gold nanoparticles, and polydopamine nanoparticles, the flexible substrate comprises one or more of thermosetting plastics such as polydimethylsiloxane and a hydrogel, and a mass percentage of the nanoparticles in the composite material is 1 to 60‰.

Preferably, the nanoparticles are carbon nanotubes, and the flexible substrate is polydimethylsiloxane.

Preferably, the mass percentage of the nanoparticles in the composite material is 5 to 30‰.

Preferably, the mass percentage of the nanoparticles in the composite material is 5 to 20‰, and preferably 5 to 10‰.

On the other hand, the present disclosure also provides a preparation method for the above-described composite material, comprising:
(1) thoroughly mixing a nanoparticle solution and a flexible substrate or a flexible substrate prepolymer, and then adding a mixture into a mold and allowing to stand to obtain a slurry; and
(2) vacuumizing the obtained slurry and curing to form a film, then taking out the mold and releasing the film from the mold, and optionally forming the resulting material to obtain said composite material.

Preferably, in step (1), the nanoparticle solution is a carbon nanotube solution, and a solvent of the solution is selected from the group consisting of water, methanol, ethanol, and dimethylformamide, and a mass fraction of the carbon nanotube solution is 1 to 10%.

Preferably, in step (1), the flexible substrate is polydimethylsiloxane, and the flexible substrate prepolymer is composed of a silicone rubber base and a silicone rubber curing agent in a mass ratio of 5 to 20:1.

Preferably, the preparation method comprises:
(1) thoroughly mixing a carbon nanotube aqueous solution with a mass fraction of 1 to 10% and a polydimethylsiloxane prepolymer, and then adding a mixture into a mold uniformly and allowing to stand for 0.5 to 2 hours until the mold is fully covered by the slurry; and
(2) vacuumizing the obtained slurry at room temperature for 0.5 to 2 hours, and curing in an oven at 60 to 80 °C for 0.5 to 2 hours to form a film, then taking out the mold and releasing the film from the mold, and optionally processing the resulting material into a desired dimension to obtain said composite material.

Finally, the present disclosure further provides a use of the composite material for preparing a material for intracellular macromolecular substance delivery and/or cell detachment.

Preferably, the macromolecular substance is selected from one or more of polysaccharide molecules, proteins, DNA, RNA, intracellular probes, therapeutic drugs, aptamers, bacteria, artificial chromosomes, or organelles.

### Advantageous effects

the present disclosure discloses a nanoparticle/flexible substrate composite material (the nanoparticles include but are not limited to carbon nanotubes, graphene, gold nanoparticles, polydopamine nanoparticles and the like; the flexible substrate includes but is not limited to PDMS, a hydrogel, etc.) which has the advantages of cheap material, easy preparation, convenient operation, low cytotoxicity, and good photothermal performance. The material is easily prepared, has very strong photothermal conversion performance, and may quickly carry out photothermal conversion under a low-intensity near-infrared laser light source; the surface roughness of the material after film formation and the quantity of the nanoparticles exposed on the surface are able to be controlled by adjusting the concentration of the nanoparticles, to finally obtain a smooth surface with specific photothermal performance without changing the original topological structure. The composite material disclosed by the present disclosure takes MCNT/PDMS as an example, and the cell membrane permeability of the cell cultured on the MCNT/PDMS may be enhanced by irradiating the near-infrared laser because of the good photothermal effect of MCNT/PDMS so that exogenous macromolecules in the solution enter the cells to realize efficient intracellular macromolecule delivery; different from the vector method, the physical method of membrane rupture is used in the present disclosure, the high-efficiency delivery of various exogenous macromolecular substances (but not limited to dextran, protein, pDNA, RNA, therapeutic drugs, intracellular probes, etc.) is realized by adjusting the laser intensity on the premise of ensuring cell viability, thereby obtaining universality and versatility for different cells (including but not limited to Hela, human embryonic fibroblast, etc.), and the delivery efficiency close to 100%; the growth and detachment of the reformed cells on the MCNT/PDMS photothermal substrate are the same as those in a culture flask by adjusting the mass fraction of the carbon nanotubes, and the modified cells may be released and harvested with high efficiency and no damage by traditional trypsin digestion, and the harvesting efficiency thereof is 90% or more; moreover, due to the flexible properties of MCNT/PDMS, MCNT/PDMS may be prepared into any size and shape of the surface to achieve high-throughput treatment of cells and achieve efficient and large-scale intracellular delivery in a short time.

### BRIEF DESCRIPTION OF THE DRAWINGS

Fig. 1 Photothermal properties of MCNT/PDMS with different contents of MCNT under different laser conditions;
Fig. 2 effect of different contents of carbon nanotubes on surface roughness of MCNT/PDMS;
Fig. 3 cell detachment and harvesting efficiency of MCNT/PDMS materials with different MCNT concentrations;
Fig. 4 changes of Hela cell membrane permeability under different laser irradiation conditions (SYTOX);
Fig. 5 changes of relative cell viability of Hela cells under different laser irradiation conditions;
Fig. 6 transfection efficiency and relative cell viability of MCNT/PDMS and Lipo2000.

### DETAILED DESCRIPTION

The present disclosure provides a composite material, wherein the composite material comprises nanoparticles and a flexible substrate, the nanoparticles comprise one or more of carbon nanotubes, graphene, gold nanoparticles, polydopamine nanoparticles, and the like, the flexible substrate comprises one or more of thermosetting plastics such as polydimethylsiloxane (PDMS) and a hydrogel, and a mass percentage of the nanoparticles in the composite material is 1 to 60‰.

In a preferred embodiment, the carbon nanotubes are multi-walled carbon nanotubes (MCNT).

In a preferred embodiment, the nanoparticles are carbon nanotubes, and the flexible substrate is polydimethylsiloxane.

The term "flexible substrate" refers to a polymer material with good elasticity, toughness, and plasticity. Due to the flexible characteristic of the substrate, a composite material doped with the photothermal nanoparticles may be formed on any surface of a well plate or a culture flask, or a composite material with a specific shape may be obtained by direct curing molding, so that macromolecule delivery may be carried out in different cell culture environments via the photoporation effect.

In a preferred embodiment, the composite material is a composition of nanoparticles and a flexible substrate.

In a more preferred embodiment, the mass percentage of the nanoparticles in the composite material is 5 to 30‰, preferably 5 to 20‰, more preferably 5 to 10‰, and most preferably 10‰.

The present disclosure also provides a preparation method for the above-described composite material, comprising:
(1) thoroughly mixing a nanoparticle solution and a flexible substrate or a flexible substrate prepolymer, and then adding a mixture into a mold and allowing to stand to obtain a slurry; and
(2) vacuumizing the obtained slurry and curing to form a film, then taking out the mold and releasing the film from the mold, and optionally forming the resulting material to obtain said composite material.

In a preferred embodiment, in step (1), the nanoparticle solution is a carbon nanotube solution, and a solvent of the solution is selected from the group consisting of water, methanol, ethanol, and dimethylformamide, and a mass fraction of the carbon nanotube solution is 1 to 10%.

In a preferred embodiment, in step (1), the flexible substrate is polydimethylsiloxane, and the flexible substrate prepolymer is obtained by mixing a silicone rubber base and a silicone rubber curing agent in a mass ratio of 5 to 20:1. The silicone rubber base is Sylgard 184 silicone rubber base, and the silicone rubber curing agent is Sylgard 184 silicone rubber curing agent.

In a preferred embodiment, the method comprises:
(1) thoroughly mixing a carbon nanotube aqueous solution with a mass fraction of 1 to 10% and a polydimethylsiloxane prepolymer, and then adding a mixture into a mold uniformly and allowing to stand for 0.5 to 2 hours until the mold is fully covered by the slurry; and
(2) vacuumizing the obtained slurry at room temperature for 0.5 to 2 hours, and curing in an oven at 60 to 80 °C for 0.5 to 2 hours to form a film, then taking out the mold and releasing the film from the mold, and optionally processing the resulting material into a disc with a desired dimension such as 0.5 to 2 cm in diameter or length to obtain said composite material.

On the other hand, the present disclosure provides a use of the above-described composite material for preparing a material for intracellular macromolecular substance delivery and/or cell detachment.

In a preferred embodiment, the mass of the above-described macromolecular substance is 0.9 to 500 kDa, and the physical dimension is 1nm to 5µm.

In a preferred embodiment, the macromolecular substance includes, but are not limited to, polysaccharide molecules (e.g., dextran), proteins (e.g., gene editing enzymes, antibodies, antigens), DNA (e.g., pDNA), RNA (e.g., mRNA, guide RNA, miRNA, siRNA), therapeutic drugs, intracellular probes (e.g., quantum dots), nanomaterials (e.g., nanoparticles and nanodevices), aptamers, bacteria, artificial chromosomes, or organelles (e.g., mitochondria), and the like.

The present disclosure further provides a use of the composite material in intracellular macromolecule delivery and cell detachment.

Finally, the prepared MCNT/PDMS is used for intracellular macromolecule delivery and harvesting of modified cells. The specific experimental method comprises:
the MCNT/PDMS material is sterilized by a sterilizing pot, cells (including but not limited to Hela cells, human embryonic fibroblast, and the like) are seeded on the MCNT/PDMS at a density of 50,000/cm², and the cells are cultured for 4 to 24 hours to enable the cells to adhere to the wall of the sample.

The cells are washed with sterile PBS, and a serum-free cell culture medium with exogenous molecules (including but not limited to plasmid DNA) is added at a final pDNA concentration of 0.005 to 0.008 µg/mL.

The cells on the sample are irradiated with a laser source in the near infrared wavelength of 808 nm at a power density of 1 to 10 W/cm² for 10 to 180 seconds.

1 to 4 hours after laser irradiation, the cells are washed with sterile PBS and trypsin is added to digest for 0.5 to 5 minutes. After the serum stopped digestion, the cells are gently blown off with a gun head until the cells completely fall off. The cells which are blown off are centrifuged and resuspended to obtain harvested cells.

The following Examples are for illustrative purposes only and do not limit the scope of the claims.

The aqueous solution of MCNT used in Examples of the present disclosure was purchased from Pioneer Nano and PDMS was purchased from Dow Corning.

### Example 1

(1) After thoroughly mixing the MCNT aqueous solution with the mass fraction of 10% and the PDMS according to the final carbon nanotube mass fraction of the composite material of 0 to 30‰, the mixture was uniformly poured into a mold and allowed to stand for 1 hour until the mold was fully covered by the slurry.
(2) The bubbles were removed by vacuumizing at room temperature for 1 hour, and then the slurry was cured in an oven at 60 to 80 °C for 1 hour. After the mold being taken out, the resultant was released from the mold, and the resulting material was cut into discs with the diameter of 1.1 cm.
(3) The solution temperature of MCNT/PDMS materials with different MCNT contents in wet state was measured by thermal imager under the laser power of 0. 6 to 3. 2 W/cm² and the irradiation time of 0 to 30 seconds, respectively.

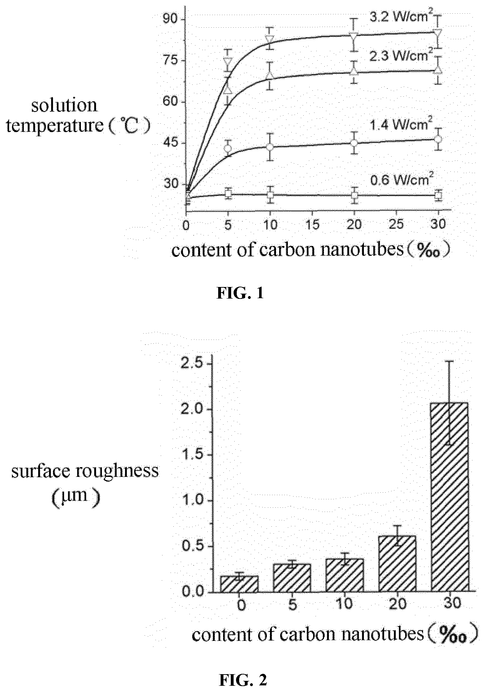

The experimental results were shown in Fig. 1, which showed that pure PDMS has no photothermal conversion ability, while after adding MCNT, 5 to 30%o MCNT/PDMS materials have good photothermal conversion performance, which provides the possibility of delivering exogenous macromolecules into cells via photoporation.

### Example 2

(1) After thoroughly mixing the MCNT aqueous solution with the mass fraction of 10% and the PDMS according to the final carbon nanotube mass fraction of the composite material of 0 to 30‰, the mixture was uniformly poured into a mold and allowed to stand for 1 hour until the mold was fully covered by the slurry.
(2) The bubbles were removed by vacuumizing at room temperature for 1 hour, and then the slurry was cured in an oven at 60 to 80 °C for 1 hour. After the mold being taken out, the resultant was released from the mold, and the resulting material was cut into discs with the diameter of 1.1 cm.
(3) The disc was thoroughly ultrasonically cleaned with water, acetone and ethanol, and dried with nitrogen for later.
(4) The surface roughness of the materials with different MCNT contents was measured by a roughness meter

The surface roughness of MCNT/PDMS composite materials with different MCNT contents was shown in Fig. 2. Fig. 2 showed that the surface roughness of MCNT/PDMS composite materials with a mass fraction of MCNT of 0 to 10%o was very small, and the surface was completely smooth. However, the surface roughness of MCNT/PDMS composite material with a mass fraction of MCNT of 20 to 30%o was increased due to the exposure of MCNT on the surface, leading to the increase in the roughness. The topological structure of the material surface might be changed by adjusting the MCNT content on the surface.

### Example 3

(1) After thoroughly mixing the MCNT aqueous solution with the mass fraction of 10% and the PDMS according to the final carbon nanotube mass fraction of the composite material of 0 to 30‰, the mixture was uniformly poured into a mold and allowed to stand for 1 hour until the mold was fully covered by the slurry. The bubbles were removed by vacuumizing at room temperature for 1 hour, and then the slurry was cured in an oven at 60 to 80 °C for 1 hour. After the mold being taken out, the resultant was released from the mold, and the resulting material was cut into discs with the diameter of 1.1 cm.
(2) A sterilizing pot with high-temperature and high-pressure steam was used to sterilize the disc. The disc was laid on the 48-well plate, and the cells (including but not limited to Hela, human embryonic fibroblast) were seeded into the wells of a 48-well plate at a density of 1 to 50,000 /well.
(3) The serum-free cell culture medium with pGFP was added, and the cells in the wells were irradiated with a laser light source with a wavelength of 808 nm at a power density of 2.3 W/cm² for 30 seconds.
(4) 1 hour after laser irradiation, the cells were washed with sterile PBS and trypsin was added to digest for 0.5 to 5 minutes. After the serum stopped digestion, the cells were gently blown off with a gun head until the cells completely fell off. The cells which were blown off were centrifuged and resuspended to obtain harvested cells. The cells on the sample were stained with DAPI dye, and the number of cells before and after detachment and after harvesting was observed.

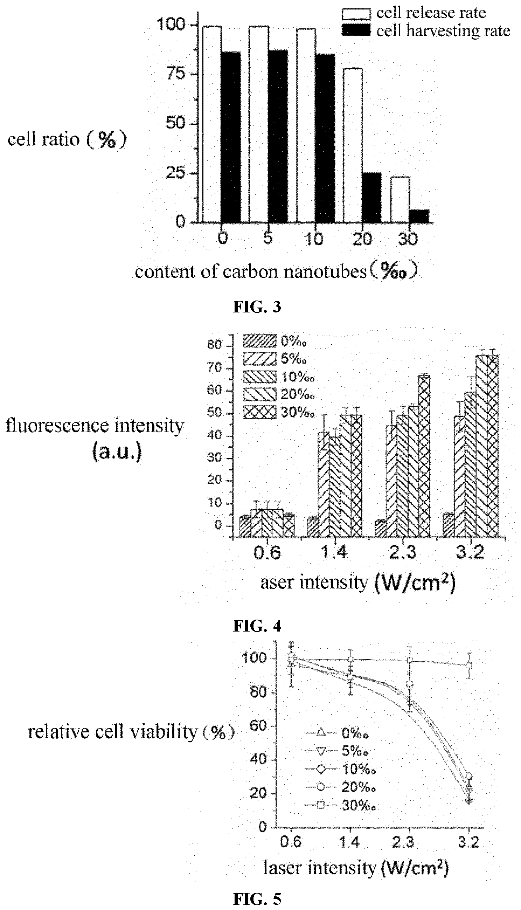

The cell detachment and harvesting efficiency of MCNT/PDMS materials with different MCNT mass fractions were shown in Fig. 3. It can be seen from Fig. 3 that the cell detachment efficiency (release rate) and harvesting efficiency (harvesting rate) of MCNT/PDMS (the mass fraction of MCNT was 0 to 10 ‰) sheets with smooth surfaces reached up to 95% and 85% respectively, which fully met the requirements of cell harvesting.

To sum up, 5 to 10‰ MCNT/PDMS has both good photothermal performance and cell detachment ability, which may be used for subsequent intracellular macromolecule delivery.

### Example 4

(1) After thoroughly mixing the MCNT aqueous solution with the mass fraction of 10% and the PDMS according to the final carbon nanotube mass fraction of the composite material of 0 to 30‰, the mixture was uniformly poured into a mold and allowed to stand for 1 hour until the mold was fully covered by the slurry. The bubbles were removed by vacuumizing at room temperature for 1 hour, and then the slurry was cured in an oven at 60 to 80 °C for 1 hour. After the mold being taken out, the resultant was released from the mold, and the resulting material was cut into discs with the diameter of 1.1 cm.
(2) A sterilizing pot with high-temperature and high-pressure steam was used to sterilize the disc. The disc was laid on the 48-well plate, and Hela cells were seeded into the wells of a 48-well plate at a density of 1 to 50,000 /well.
(3) The serum-free cell culture medium with SYTOX (a dye that may only pass through the damaged cell membrane, and the higher the fluorescence intensity, the better the cell membrane permeability) was added, and the cells in the wells were irradiated with a laser source with a wavelength of 808 nm at a power density of low intensity of 0.6 to 3.2 W/cm² for 30 seconds.
(4) 1 to 4 hours after laser irradiation, the medium was changed to complete medium to continue cell culture.
(5) 2 to 4 hours after laser irradiation, the living cells were stained with Calcein, and then the fluorescence was observed by a fluorescence microscope.
(6) 48 hours after laser irradiation, the relative cell viability was detected by CCK-8 kit (cell viability test kit).

The changes of Hela cell membrane permeability (SYTOX) under different laser irradiation conditions were shown in Fig. 4. It can be seen from Fig. 4 that pure PDMS did not change the permeability of cell membrane, but 5 to 30 ‰ MCNT/PDMS might change the permeability of cell membrane obviously at 1.4 to 3.2 W/cm², and the cell permeability increased with the increase of MCNT content. The viability of Hela cells under different lase irradiation conditions was shown in Fig. 5. It can be seen from Fig. 5 that the relative cellular viability of cells may be 80% or more with the laser intensity of 0.6 to 2.3 W/cm². This demonstrated the feasibility of improving cell membrane permeability and delivering exogenous macromolecules by irradiating MCNT/PDMS with near-infrared laser irradiation.

To sum up, when the power was 2.3W/ cm² and the irradiation time was 30 seconds, the cell membrane permeability and cell viability of the disc with MCNT content of 10 %o were better. By changing the laser irradiation conditions, cells excellent in both viability and membrane permeability might be obtained.

### Example 5

(1) After thoroughly mixing the MCNT aqueous solution with the mass fraction of 10% and the PDMS according to the final carbon nanotube mass fraction of the composite material of 10‰, the mixture was uniformly poured into a mold and allowed to stand for 1 hour until the mold was fully covered by the slurry. The bubbles were removed by vacuumizing at room temperature for 1 hour, and then the slurry was cured in an oven at 60 to 80 °C for 1 hour. After the mold being taken out, the resultant was released from the mold, and the resulting material was cut into discs with the diameter of 1.1 cm.
(2) A sterilizing pot with high-temperature and high-pressure steam was used to sterilize the disc. The disc was laid on the 48-well plate, and Hela cells and human embryonic fibroblast cells were seeded into the wells of a 48-well plate at a density of 1 to 50,000 /well.
(3) The serum-free cell culture medium with pGFP was added, and the cells in the wells were irradiated with a laser light source with a wavelength of 808 nm at a power density of 2.3 W/cm² for 30 seconds.
(4) 1 to 4 hours after laser irradiation, the medium was changed to complete medium to continue cell culture.
(5) 48 hours after laser irradiation, the nuclei were stained with DAPI, and then the expression of green fluorescent protein was observed by the fluorescence microscope.

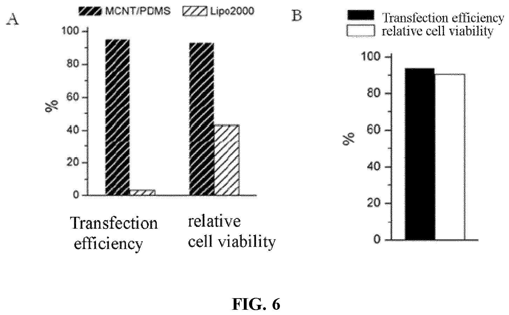

The transfection efficiency and relative cell viability of pGFP delivered to Hela cells and human embryonic fibroblasts under a laser irradiation condition with 2.3 W/cm² and 30 seconds were shown in Fig. 6 A and B, respectively. It can thus be seen that the method for delivering macromolecular substances into cells based on the composite material of the present disclosure is highly versatile for cell types, and it can be seen from B in Fig. 6 that the transfection efficiency for difficult-to-transfect cells such as human embryonic fibroblasts might reach 93% or more, and the good viability of the cells was maintained.

### Comparative Example 1

The conditions of Comparative Example 1 and Example 5 are the same, except that commercial Lipo2000 was used as transfection reagent in Comparative Example 1 instead of MCNT/PDMS composite material, and only Hela cells were tested.

The transfection efficiency and relative cell viability of pGFP delivered to Hela cells under a laser irradiation condition with 2.3 W/cm² and 30 seconds were shown in Fig. 6A. As can be seen from that figure, under the same conditions, the transfection efficiency of Lipo2000 to the primary cell was less than 10%, and the good activity of cells was not maintained.

## Claims

1. A composite material, wherein the composite material comprises nanoparticles and a flexible substrate, the nanoparticles comprise one or more of carbon nanotubes, graphene, gold nanoparticles, and polydopamine nanoparticles, the flexible substrate comprises one or more of polydimethylsiloxane and a hydrogel, and a mass percentage of the nanoparticles in the composite material is 1 to 60‰.

2. The composite material according to claim 1, wherein the nanoparticles are carbon nanotubes, and the flexible substrate is polydimethylsiloxane.

3. The composite material according to claim 1, wherein the mass percentage of the nanoparticles in the composite material is 5 to 30‰.

4. The composite material according to any one of claims 1 to 3, wherein the mass percentage of the nanoparticles in the composite material is 5 to 20‰, and preferably 5 to 10‰.

5. A preparation method for the composite material according to any one of claims 1 to 4, comprising:
(1) thoroughly mixing a nanoparticle solution and a flexible substrate or a flexible substrate prepolymer, and then adding a mixture into a mold and allowing to stand to obtain a slurry; and
(2) vacuumizing the obtained slurry and curing to form a film, then taking out the mold and releasing the film from the mold, and optionally forming the resulting material to obtain said composite material.

6. The preparation method for the composite material according to claim 5, wherein in step (1), the nanoparticle solution is a carbon nanotube solution, and a solvent of the solution is selected from the group consisting of water, methanol, ethanol, and dimethylformamide, and a mass fraction of the carbon nanotube solution is 1 to 10%.

7. The preparation method for the composite material according to claim 5, wherein in step (1), the flexible substrate is polydimethylsiloxane, and the flexible substrate prepolymer is a composition of a silicone rubber base and a silicone rubber curing agent in a mass ratio of 5 to 20:1.

8. The preparation method for the composite material according to claim 5, comprising:
(1) thoroughly mixing a carbon nanotube aqueous solution with a mass fraction of 1 to 10% and a polydimethylsiloxane prepolymer, and then adding a mixture into a mold uniformly and allowing to stand for 0.5 to 2 hours until the mold is fully covered by the slurry; and
(2) vacuumizing the obtained slurry at room temperature for 0.5 to 2 hours, and curing in an oven at 60 to 80 °C for 0.5 to 2 hours to form a film, then taking out the mold and releasing the film from the mold, and optionally processing the resulting material into a desired dimension to obtain said composite material.

9. Use of the composite material according to any one of claims 1 to 4 for preparing a material for intracellular macromolecular substance delivery and/or cell detachment.

10. The use according to claim 9, wherein the macromolecular substance is selected from one or more of polysaccharide molecules, proteins, DNA, RNA, intracellular probes, therapeutic drugs, aptamers, bacteria, artificial chromosomes, or organelles.
